# EUROPEAN PATENT APPLICATION

(11) **EP 3 470 895 A1**
(43) Date of publication of application: **17.04.2019**
(21) Application number: 17195597.4
(22) Date of filing: 10.10.2017
(51) Int. Cl.: G02B 6/00, C03C 25/70, B08B 3/04, A61B 18/22

(54) **TREATING AN OPTICAL WAVEGUIDE**

(71) Applicant: Koninklijke Philips N.V., 5656 AE Eindhoven (NL)
(72) Inventor: BOAMFA, Marius Iosif, 5656 AE Eindhoven (NL); VERHAGEN, Rieko, 5656 AE Eindhoven (NL); JURNA, Martin, 5656 AE Eindhoven (NL); MOESKOPS, Bastiaan Wilhelmus Maria, 5656 AE Eindhoven (NL); JOHNSON, Mark Thomas, 5656 AE Eindhoven (NL); THUMMA, Kiran Kumar, 5656 AE Eindhoven (NL)
(74) Representative: de Haan, Poul Erik

(57) **Abstract**

There is provided a method (500) of treating an optical waveguide. The method comprises applying (502) the volume of ferrofluid to the optical waveguide, and applying (504) a magnetic field to the volume of ferrofluid to move the volume of ferrofluid at least partially along a length of the optical waveguide. A personal care device and a system are also disclosed.

## Description

### TECHNICAL FIELD OF THE INVENTION

The invention relates to an optical waveguide and, more particularly, to a method, device and system for treating an optical wave.

### BACKGROUND TO THE INVENTION

A shaving device has been proposed in WO 2014/143670 that makes use of laser light. In particular, a laser light source is provided that is configured to generate laser light having a wavelength selected to target a predetermined chromophore to effectively cut a hair shaft. An optical fibre is located on a shaving portion of the device that is positioned to receive the laser light from the laser light source at a proximal end, conduct the laser light from the proximal end toward a distal end, and emit the light out of a cutting region of the optical fibre and toward hair when the cutting region is brought in contact with the hair.

Over time, use of such a shaving device may cause the optical fibre to become dirty. For example, debris, such as pieces of cut hair, dust or dirt may accumulate on the optical fibre. Such dirt may adversely affect the cutting ability of the cutting device, thereby reducing the effectiveness of the device.

Furthermore, debris built up on the optical fibre may be caused to heat up and eventually burn during use of the shaving device. Consequently, the optical fibre may be caused to heat up during use. If the temperature of the optical fibre increased too much, or too rapidly, then the optical fibre may be damaged.

Conventional optical fibre cleaning methods include wiping the dirt off the optical fibre, for example by using a cloth. However, dues to the fragility of the optical fibre, such cleaning methods may cause damage to the optical fibre.

### SUMMARY OF THE INVENTION

Treating (e.g. cleaning) an optical fibre regularly may lengthen the life of the optical fibre and the shaving or cutting device in which it is installed. Furthermore, a cleaner optical fibre is likely to be more efficient at cutting hair than one on which dirt and debris has built up. Thus, an improved method for treating an optical fibre may improve the hair cutting experience for a user, and may improve the lifetime of the user's device.

Thus, there is a need for a method of treating an optical fibre which reduces the likelihood of damaging the optical fibre.

According to a first aspect, there is provided a method of treating an optical waveguide, the method comprising applying the volume of ferrofluid to the optical waveguide; and applying a magnetic field to the volume of ferrofluid to move the volume of ferrofluid at least partially along a length of the optical waveguide. By applying a volume of ferrofluid to the optical waveguide, it is possible to treat and clean the optical waveguide without applying direct pressure to the waveguide, which may result in the damage. The ferrofluid can be moved along the optical waveguide by controlling a magnetic field to manipulate the volume of ferrofluid in an intended manner.

According to some embodiments, applying a magnetic field may comprise applying a magnetic field generated by at least one of a permanent magnet and an electromagnet.

The ferrofluid may comprise a binder fluid. The binder fluid may, in some embodiments, comprise an oil-based binder fluid.

In some embodiments, the ferrofluid may comprise a chemical for providing a cleaning effect to the optical waveguide. In some embodiments, the chemical may dissolve pieces of hair that have accumulated on the optical waveguide.

The ferrofluid may comprise an additive for causing an abrasive effect when the additive is moved into contact with the optical waveguide.

The method may, in some embodiments, further comprise causing the magnetic field to move at least one of longitudinally relative to the optical waveguide and laterally relative to the optical waveguide.

The method may further comprise displacing at least a portion of the volume of ferrofluid from the optical waveguide. Such displacement may be used to remove some or all of the ferrofluid from the optical waveguide once the optical waveguide has been treated.

According to a second aspect, there is provided a personal care device comprising an optical waveguide having a sidewall, wherein a portion of the sidewall forms a cutting face for contacting hair. The personal care device further comprises a magnetic field source configured to generate a magnetic field for engaging a volume of ferrofluid present on the optical waveguide. Thus, the personal care device may include the means for manipulating the ferrofluid.

The personal care device may, in some embodiments, further comprise a magnetic field movement mechanism for causing the magnetic field to move relative to the optical waveguide.

In some embodiments, the personal care device may further comprise an applicator for applying a volume of ferrofluid to the optical waveguide.

The personal care device may further comprise a fluid displacement device for displacing at least a portion of the volume of the ferrofluid from the optical waveguide.

According to a third aspect, there is provided a system comprising a personal care device, the personal care device comprising an optical waveguide having a sidewall, wherein a portion of the sidewall forms a cutting face for contacting hair. The system further comprises a docking unit for receiving at least a portion of the personal care device. The docking unit comprises a magnetic field generator for generating a magnetic field to engage a volume of ferrofluid disposed on the optical waveguide of the personal care device. With this arrangement, the components used to manipulate the ferrofluid are included in the docking unit rather than in the personal care device. Therefore, the personal care device (or components thereof) may be disposable or replaceable.

In some embodiments, the docking unit may further comprise a magnetic field movement mechanism for causing the magnetic field to move relative to the optical waveguide.

The docking unit may further comprise an applicator for applying a volume of ferrofluid to the optical waveguide.

The docking unit may in some embodiments, further comprise a fluid displacement device for displacing at least a portion of the volume of the ferrofluid from the optical waveguide.

These and other aspects of the invention will be apparent from and elucidated with reference to the embodiments described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a block diagram of a hair cutting device according to embodiments of the invention;
Fig. 2 is a pair of schematic drawings showing different views of an exemplary hair cutting device;
Fig. 3 is a schematic illustration of an example of an arrangement for manipulating a ferrofluid on an optical waveguide;
Fig. 4 is a schematic illustration of a further example of an arrangement for manipulating a ferrofluid on an optical waveguide;
Fig. 5 is a flowchart of an example of a method of treating an optical waveguide;
Fig. 6 is a flowchart of a further example of a method of treating an optical waveguide;
Fig. 7 is a flowchart of a further example of a method of treating an optical waveguide;
Fig. 8 is a schematic illustration of an example of a personal care device;
Fig. 9 is a schematic illustration of an example of a portion of the personal care device of Fig. 8;
Fig. 10 is a schematic illustration of an example of a personal care system; and
Fig. 11 is a schematic illustration of an example of a portion of the personal care system of Fig. 10.

### DETAILED DESCRIPTION OF EMBODIMENTS

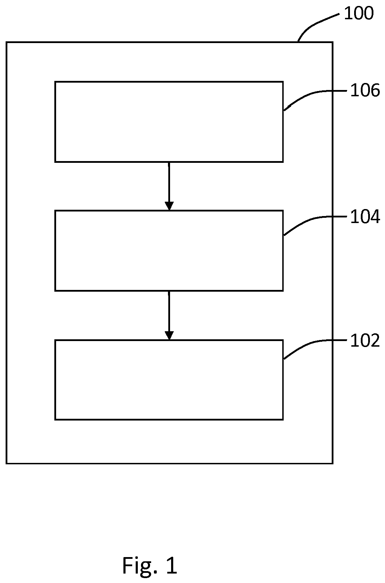

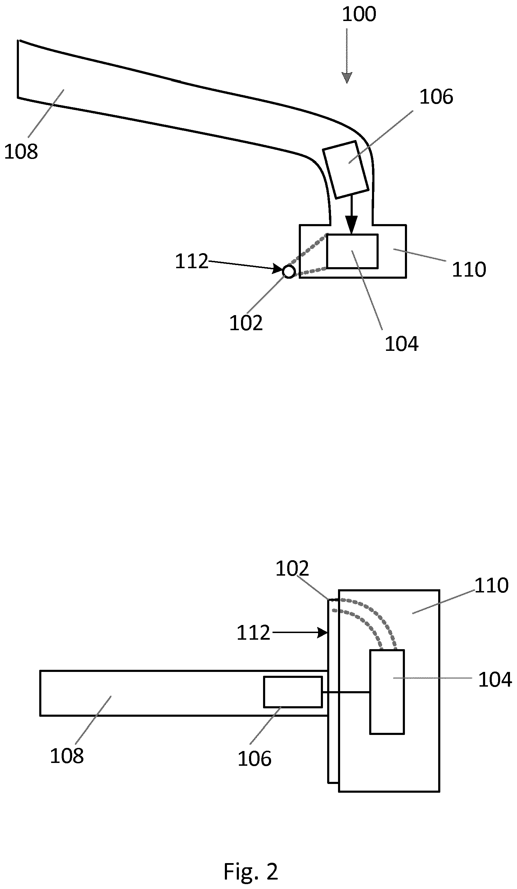

Fig. 1 is a block diagram of a hair cutting device 100. Fig. 2 shows a hair cutting device 100 in the form of a handheld razor according to an exemplary embodiment of the invention. The hair cutting device 100 is for cutting (e.g. shaving) hair on a body of a subject. The subject may be a person or an animal. The hair may be facial hair (i.e. hair on the subject's face), or hair on the subject's head or other part of their body (legs, chest, etc.).

The hair cutting device 100 comprises a cutting element 102 that enables hair to be cut as the hair cutting device 100 is moved over the skin of a subject. The cutting element 102 is an optical waveguide 102 that is arranged on the hair cutting device 100 so that the optical axis of the optical waveguide 102 (i.e. the line along which light typically propagates through the optical waveguide 102) is generally perpendicular to the direction in which the hair cutting device 100 is moved so that hairs contact the sidewall of the optical waveguide 102 (the sidewall corresponding to the long edge of the optical waveguide 102) as the hair cutting device 100 is moved across the skin of the subject. In some embodiments, the optical waveguide 102 is an optical fibre, although those skilled in the art will be aware of other types of optical waveguide that can be used according to the invention, such as a slab waveguide, a strip waveguide or a photonic crystal waveguide. An optical fibre comprises a core, and in some embodiments also comprises a cladding, which may or may not fully encompass the core (e.g. part of the core may be exposed). The optical waveguide 102 may form part of a cutting assembly of the hair cutting device 100. The cutting assembly may, in some embodiments, be a detachable and/or replaceable component, and may be designed to be replaced as the optical waveguide 102, or other components of the cutting assembly, become worn or damaged.

A light source 104 is provided in the hair cutting device 100 that generates laser light at one or more specific wavelengths. The light source 104 is optically coupled to the optical waveguide 102 so that the laser light generated by the light source 104 is coupled into the optical waveguide 102 (and specifically coupled into at least one end of the optical waveguide 102 so that the laser light propagates through the optical waveguide 102).

The light source 104 is configured to generate laser light at one or more specific wavelengths that can be used to cut or burn through hair. In particular, each wavelength corresponds to the wavelength of light absorbed by a chromophore that is found in hair. As is known, a chromophore is the part of a molecule that provides the molecule with its colour. Thus, the laser light will be absorbed by the chromophore and converted into heat which will melt or burn the hair or otherwise destroy the bonds in the molecules of the hair, and it is this melting or burning that provides the cutting action of the hair cutting device 100.

Suitable chromophores that can be targeted by the laser light generated by the light source 104 include, but are not limited to, melanin, keratin and water. Suitable wavelengths of laser light that can be used include, but are not limited to, wavelengths selected from the range 380 nm (nanometres) to 500 nm and 2500 nm to 3500 nm. Those skilled in the art will be aware of the wavelengths of light that are absorbed by these chromophores, and thus also the specific wavelengths of light that the light source 104 should generate for this purpose, and further details are not provided herein.

In some embodiments the light source 104 can be configured to generate laser light at a plurality of wavelengths (either simultaneously or sequentially), with each wavelength being selected to target a different type of chromophore. This can improve the cutting action of the optical waveguide 102 since multiple types of molecules in the hair may be burnt using the laser light. Alternatively multiple light sources 104 can be provided that each generate laser light at a respective wavelength. The multiple light sources may be coupled to a single optical waveguide, or each light source 104 can be coupled to a respective optical waveguide 102 to provide multiple cutting elements 102 in the device 100.

The hair cutting device 100 may also comprise a control unit 106 that controls the operation of the hair cutting device 100, and in particular may be connected to the light source 104 to control the activation and deactivation of the light source 104 (and in some embodiments control the wavelength and/or intensity of the light generated by the light source 104). The control unit 106 may activate and deactivate the light source 104 in response to an input from a user of the hair cutting device 100. The control unit 106 can comprise one or more processors, processing units, multi-core processors or modules that are configured or programmed to control the hair cutting device 100.

As noted above, Fig. 2 shows a hair cutting device 100 that is in the form of a handheld wet razor. Fig. 2 shows a side view and a bottom view of the razor 100. The razor 100 comprises a handle 108 for the subject (or other user of the device 100) to hold, and a head portion 110 that includes the cutting element 102 (optical waveguide/fibre). As shown, the optical waveguide 102 is arranged along an edge of the head portion, and a part of the optical waveguide 102 forms (or corresponds to) a cutting face 112. The cutting face 112 is the part of the optical waveguide 102 that is intended to come into contact with hair as the hair cutting device 100 is moved across the skin of the subject. A light source 104 and control unit 106 are shown as being incorporated into the head portion 110 and handle 108 respectively, but it will be appreciated that the positions of these components in the hair cutting device 100 as shown in Fig. 2 is not limiting. Likewise it will be appreciated that the embodiment shown in Fig. 2 is merely an example, and the invention can be incorporated or used in any type of hair cutting device 100 that comprises an optical waveguide cutting element 102 as described herein.

As is known, the optical waveguide 102 acts as a waveguide for the light coupled from the light source 104 through the occurrence of total internal reflection, since the refractive index of air is lower than that of the optical waveguide 102. However, if an object that has a refractive index higher than the optical waveguide 102 is put into contact with the optical waveguide 102, then the total internal reflection is 'frustrated' and light can couple from the optical waveguide 102 into that object. Thus, in order for light to be coupled into a hair from the optical waveguide 102 (to provide the cutting action according to the invention), the optical waveguide 102 should preferably have the same or a lower refractive index than hair at the point at which the hair contacts the optical waveguide 102. Thus, the optical waveguide 102 should preferably have the same or a lower refractive index than hair at least at the cutting face 112 portion of the optical waveguide 102. Preferably the refractive index of the optical waveguide 102 at the cutting face 112 is the same as that of hair since that provides the best coupling of light from the optical waveguide 102 to the hair. Light may still be able to couple from the optical waveguide 102 into an object (e.g. a hair) brought into contact with the cutting face 112 of the optical waveguide even if the refractive index of the optical waveguide is higher than that of the object, due to a high numerical aperture in the cutting face.

Pieces of hair that have been cut by the optical waveguide 102 may rest on the optical waveguide, or may become stuck to the optical waveguide, for example by a shaving liquid, such water or shaving gel applied to the user's face. Particles of dust, dirt and other debris may also accumulate on the optical waveguide 102 and, as noted above, the high temperatures experienced by the cutting face of the optical waveguide may cause such debris to burn. In order to assist a user in removing such debris from the optical waveguide 102, the present invention involves the use of a ferrofluid. A ferrofluid is a substance that becomes magnetized in the presence of a magnetic field. A ferrofluid is a colloidal suspension which includes ferromagnetic, or ferrimagnetic, particles suspended in a carrier fluid. The particles may, in some examples, be nanoparticles. The carrier fluid may be water, or some other liquid, such as an organic solvent.

In general, the invention relates to the use of a ferrofluid to treat an optical waveguide. Due to the inherent properties of a ferrofluid (i.e. its magnetization in a magnetic field), a volume of ferrofluid, such as a droplet of ferrofluid, can be manipulated by controlling a magnetic field acting on the ferrofluid.

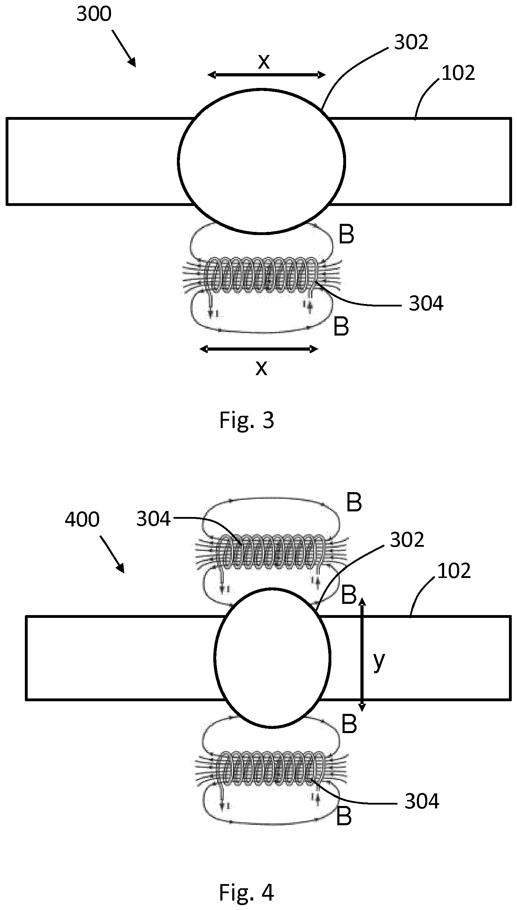

Fig. 3 is a schematic illustration of an arrangement 300 showing an example of how a ferrofluid may be used to treat an optical waveguide in accordance with embodiments of the invention. In the arrangement 300 of Fig. 3, a portion of the optical waveguide 102 is shown. It will be appreciated that the optical waveguide 102 may form a part of the cutting device 100 discussed above. The cutting device 100 is omitted from Fig. 3 for clarity. A volume of ferrofluid 302 is present on the optical waveguide 102. Any volume of ferrofluid may be applied to the optical waveguide 102. However, it will be apparent that a volume sufficient to extend around the circumference of the optical waveguide 102 may result in a more efficient treatment process. If the volume of ferrofluid applied to the optical waveguide 102 is too large, then it may be more difficult to manipulate the ferrofluid using an applied magnetic field. Therefore, in some embodiments, a droplet of ferrofluid may be applied to the optical waveguide 102.

A magnetic field source 304 is provided proximal to the optical waveguide 102, and is configured to generate a magnetic field *B.* The magnetic field source 304 is positioned such that the generated magnetic field *B* is able to interact with the volume of ferrofluid 302. In the example shown in Fig. 3, the magnetic field source 304 comprises an electromagnetic coil capable of generating a magnetic field when a current *I* is passed through the coil. In other examples, alternative magnetic field sources may be used, such as one or more permanent magnets. While, in Fig. 3, the magnetic field source 304 is shown to one side of the optical waveguide 102, it will be appreciated that the magnetic field source may extend partially around the optical waveguide, for example around half of the circumference of the optical waveguide.

The magnetic field *B* shown in the arrangement 300 of Fig. 3 can be manipulated in such a way that it moves along an optical axis of the optical waveguide 102, for example in the direction *x* indicated by the arrows. Manipulation of the magnetic *field B* may be achieved in a number of ways. In some examples, the magnetic field *B* may be generated by a magnetic field source 304 (e.g. a coil) extending along only a portion of a length of the optical waveguide 102. The magnetic field source 304 may be moveable along the length of the optical waveguide 102, so that the magnetic field *B* can be moved in the direction *x*. In this way, the magnetic field source 304 and the magnetic field *B* it generates can be moved in either direction along the length of the optical waveguide 102. The magnetic field *B* interacts with the volume of ferrofluid 302 and may, for example, cause the ferrofluid to be attracted towards (or repelled away from) the magnetic field source 304. By moving the magnetic field source 304 in an appropriate manner (e.g. along the length of the optical waveguide), the volume of ferrofluid 302 can be caused to move along the optical waveguide 102. In other examples, a magnetic field source 304 may extend along the length of the optical waveguide 102, for example as a series of electromagnetic coils. With such an arrangement, portions of the magnetic field source 304 may be independently controlled and actuated (e.g. by switching on a first coil, then switching off the first coil and switching on a second, adjacent coil, then switching off the second coil and switching on a third coil, and so on), to cause the volume of ferrofluid to be moved along the optical waveguide 102. In this arrangement, the magnetic field source 304 does not move relative to the optical waveguide 102, so a mechanism to move the magnetic field source is not included.

Parameters of the magnetic field *B* and, therefore, parameters of the magnetic field source may be selected or controlled such that the volume of ferrofluid may be moved along the optical waveguide, but is not removed from the waveguide. In other words, the magnetic field may be controlled such that the force exerted on the ferrofluid in a direction away from the optical waveguide is not stronger than the surface tension causing the ferrofluid to remain in contact with the optical waveguide.

As the ferrofluid moves along the optical waveguide 102, particles of cut hair, dust and other debris may be removed from the optical waveguide and may accumulate in the volume of ferrofluid 302. Causing the volume of ferrofluid 302 to move along the length of the optical waveguide 102 repeatedly may result in the optical waveguide becoming cleaner, with fewer particles of debris remaining on the waveguide. As explained below, once the ferrofluid has been moved along the optical waveguide 102, at least a portion of the volume of ferrofluid 302 may be removed from the optical waveguide, along with debris that has been removed from the waveguide.

Fig. 4 is a schematic illustration of an arrangement 400 showing an example of how a ferrofluid may be used to treat an optical waveguide in accordance with embodiments of the invention. In the arrangement 400 of Fig. 4, a portion of the optical waveguide 102 is shown with a volume of ferrofluid 302 disposed on the optical waveguide. In the arrangement 400, a first magnetic field source 304 is provided on one side of the optical waveguide 102, and a second magnetic field source 306 is provided on an opposing side of the optical waveguide. In some embodiments, the first and second magnetic field sources 304, 306 may form part of the same magnetic field source, but may be individually operable and controllable. Using the arrangement 400 of Fig. 4, the magnetic field sources 304, 306 may be actuated (i.e. caused to generate a magnetic field *B* alternately, or simultaneously but at different strengths. In other words, the first magnetic field source 304 may generate a magnetic field *B* during a first time period *t₁* while the second magnetic field source 306 does not generate a magnetic field. The first magnetic field source 304 may then be de-activated (e.g. switched off) so that it does not generate a magnetic field for a timer period *t₂*, during which period the second magnetic field source 306 may generate a magnetic field *B.* Applying an alternating magnetic field in this way may cause the volume of ferrofluid 302 to move in an oscillatory fashion between the first and second magnetic field sources, in a direction *y* indicated by the arrow in Fig. 4. By causing the ferrofluid to move in this way, dirt and debris stuck to the optical waveguide 102 may be released and suspended in the ferrofluid.

In a further example, a magnetic field source 304, 306 may be disposed at one or more positions along the optical waveguide 102. For example, a magnetic field source may be positioned at a first end of the optical waveguide. In this way, a volume of ferrofluid deposited at a second end (i.e. opposite to the first end) may be attracted to, by the magnetic field, to the magnetic field source at the first end. In some examples, a magnetic field source may be positioned at each end of the optical waveguide 102. In this way, alternately causing each of the magnetic field sources to generate a magnetic field will cause a volume of ferrofluid to travel along the length of the optical waveguide in a first direction, then back in the opposite direction, and so on.

In a further example, the arrangements 300 and 400 may be combined such that the volume of ferrofluid 302 may be moved in an oscillatory manner (e.g. vibrated) in the *y* direction while it is moved along the length of the optical waveguide 102 in the *x* direction. In this way, the effectiveness of the ferrofluid in removing debris from the optical waveguide may be further improved.

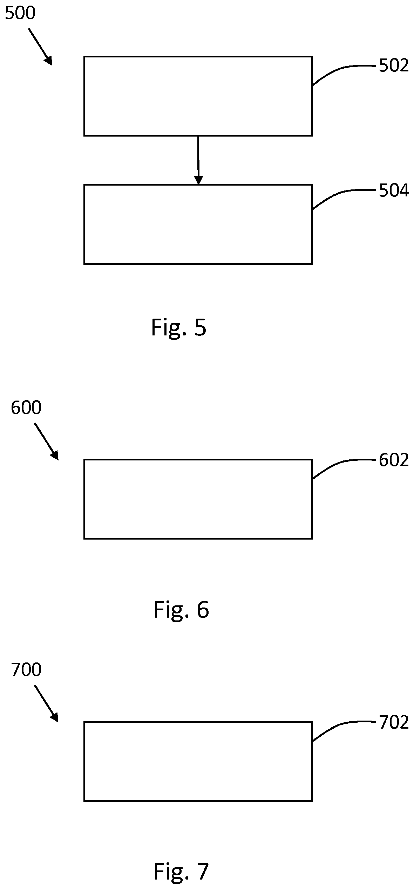

According to a first aspect, a method is provided. Fig. 5 is a flowchart of an example of a method 500 of treating an optical waveguide 102. The method comprises, at step 502, applying the volume of ferrofluid 302 to the optical waveguide 102. At step 504, the method 500 comprises applying a magnetic field to the volume of ferrofluid 302 to move the volume of ferrofluid at least partially along a length of the optical waveguide 102.

The magnetic field may be applied to the volume of ferrofluid by using a magnetic field source such as the source 304 and/or the source 306, as discussed above. The volume of ferrofluid may, in some embodiments, be a single droplet. The ferrofluid may be applied to the optical waveguide 102 using any suitable dispensing means. For example, the ferrofluid may be applied manually using a device, such as a dropper or pipette. Alternatively, the ferrofluid may be applied using an automatic device configured, for example, to deposit a defined amount (e.g. volume) of ferrofluid from a ferrofluid reservoir onto the optical waveguide 102.

As noted above, the magnetic field may be generated using one or more various sources. In some embodiments, the step of applying (504) a magnetic field may comprise applying a magnetic field generated by at least one of a permanent magnet and an electromagnet. In the case of an electromagnet being used as the magnetic field source, one or more electromagnetic coils may be used. In some embodiments, a single coil may be movable along the length of the optical waveguide while, in other embodiment, one or more coils which are stationary with respect to the optical waveguide may be positioned along the length of the optical waveguide. In embodiments in which one or more stationary coils are implemented, each coil may be selectively controlled to generate a magnetic field of a particular strength at a particular time and for a particular duration.

In addition to ferromagnetic particles, the ferrofluid may include one or more additives for providing additional benefits. In some embodiments, the ferrofluid may comprise a binder fluid. A binder fluid may be included to improve the binding effect of the ferrofluid to the optical waveguide. A volume of ferrofluid which includes such a binder fluid may deposit a portion of the binder fluid onto the optical waveguide as the volume of ferrofluid moves along the length of the optical waveguide. The binder fluid may be selected such that the coating, or film, deposited on the optical waveguide improves the cutting effectiveness of the optical waveguide. For example, a binder fluid may be chosen which, when deposited on the optical waveguide, helps to initiate the hair cutting process. Such an effect may, for example, be achieved using an oil-based binder fluid.

In some embodiments, the ferrofluid may comprise a chemical, for example in addition to, or instead of, the binder fluid. The chemical may, in some examples, provide a cleaning effect to the optical waveguide. In one embodiment, the ferrofluid may comprises a chemical for targeting a chromophore present in hair, such as keratin. For example, the chemical may break down or dissolve keratin. In this way, the chemical may serve to dissolve pieces of hair disposed on (e.g. stuck to) the optical waveguide, thereby further improving the cleaning effect of the ferrofluid.

The ferrofluid may, in some embodiments, comprise an additive for causing an abrasive effect when the additive is moved into contact with the optical waveguide. For example, the ferrofluid may comprise micro-particles. An abrasive additive such as micro-particles may serve to knock or rub against the optical waveguide as the ferrofluid moves over the optical waveguide. In some examples, particles (e.g. micro-particles) of an abrasive additive may be caused to move around within the volume of ferrofluid as the volume of ferrofluid oscillates (e.g. vibrates) on the optical waveguide due to the magnetic field, as exemplified in the arrangement 400 of Fig. 4.

The ferrofluid may comprise one or more of the fluids, chemicals and additives discussed above, and/or one or more other additives.

Fig. 6 is a flowchart of an example of a method 600 of treating an optical waveguide, such as the optical waveguide 102. The method 600 may comprise one or more of the steps of the method 500 above. The method 600 may comprise, at step 602, causing the magnetic field to move at least one of longitudinally relative to the optical waveguide and laterally relative to the optical waveguide. In other words, the magnetic field may be caused to move in the *x* direction (see Fig. 3) and/or in the *y* direction (see Fig. 4). In order to move the magnetic field, the magnetic field source may be caused to move at least one of longitudinally relative to the optical waveguide and laterally relative to the optical waveguide.

Fig. 7 is a flowchart of an example of a method 700 of treating an optical waveguide, such as the optical waveguide 102. The method 700 may comprise one or more of the steps of the methods 500, 600 above. The method 700 may comprise, at step 702, displacing at least a portion of the volume of ferrofluid from the optical waveguide. Some or all of the volume of ferrofluid may be displaced from the optical waveguide 102 once it is has been used to treat (e.g. clean) the optical waveguide. For example, after a defined time of moving the ferrofluid along the optical waveguide, the ferrofluid (or a portion thereof) may be removed. Debris removed from the optical waveguide by the ferrofluid may also be removed by displacing the volume of ferrofluid.

Displacement of at least a portion of the volume of ferrofluid may be achieved in various ways. For example, some or all of the ferrofluid may be displayed (e.g. removed) from the optical waveguide using a fluid-flow device arranged to direct a fluid (e.g. a stream, jet or curtain of air, water or another gas or liquid) towards the volume of ferrofluid so as to remove an amount of the ferrofluid from the optical waveguide. It will be appreciated that, in some scenarios, it may be desirable for an amount of the volume of ferrofluid to remain on the optical waveguide, for example to coat a surface of the optical waveguide to improve the effectiveness of the coupling of light from the waveguide into hair to be cut. As such, while the device to displace the ferrofluid may, in some embodiments, be directed or aimed at the entire length of the optical waveguide, in other embodiments, the device may be directed towards a particular portion of the optical waveguide.

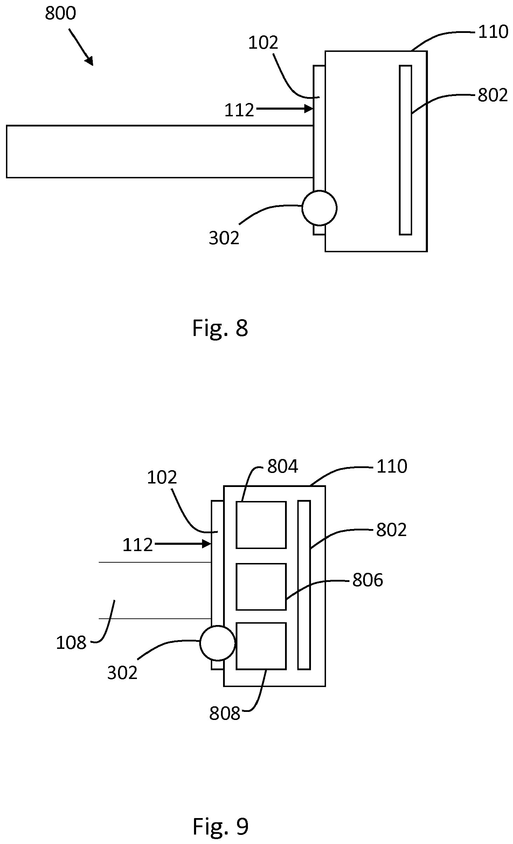

According to another aspect, the invention relates to a personal care device, such as the hair cutting device 100 discussed above. Fig. 8 is a schematic illustration of an example of a personal care device 800. The personal care device 800 comprises an optical waveguide 102 having a sidewall, wherein a portion of the sidewall forms a cutting face 112 for contacting hair. The personal care device 800 further comprises a magnetic field source 802 configured to generate a magnetic field for engaging a volume of ferrofluid 302 present on the optical waveguide 102. In some embodiments, the personal care device 800 may include a handle portion 108 and a head portion 110. In the embodiment shown in Fig. 8, the magnetic field source 802 is located in the head portion 110. However, it will be appreciated that, in other embodiments, the magnetic field source 802 may be located elsewhere in the personal care device 800, such as in the handle portion 108.

The magnetic field source 802 is shown in Fig. 8 to extend substantially along the length of the optical waveguide 102. However, it will be appreciated that the magnetic field source 802 may be sized, positioned and/or configured to focus a magnetic field on a particular portion of the optical waveguide, and may be moveable (e.g. in the direction of the optical axis of the optical waveguide), as discussed above.

Fig. 9 is a schematic illustration of a further example of the personal care device 800. According to various embodiments, the personal care device 800 may be provided with one or more additional or alternative components. In some embodiments, the personal care device 800 may comprise a magnetic field movement mechanism 804 for causing the magnetic field to move relative to the optical waveguide 102. The magnetic field movement mechanism 804 may move the magnetic field source 802, or cause the magnetic field source to move, for example across or along the optical waveguide. Alternatively, the magnetic field movement mechanism 804 may manipulate the magnetic field itself, for example by directing the magnetic field and/or controlling the strength of the magnetic field at particular places along the optical waveguide.

The personal care device 800 may, in some embodiments, comprise an applicator 806 for applying a volume of ferrofluid 302 to the optical waveguide. The applicator, or ferrofluid applicator, 806 may comprise any mechanism suitable for applying a volume, or multiple volumes, of ferrofluid (e.g. in the form of a droplet) to a particular location of the optical waveguide. The applicator 806 may, for example, comprise a spray mechanism for spraying the ferrofluid onto the optical waveguide 102, a nebuliser device for depositing the ferrofluid on the optical waveguide, a mechanism similar to, or based on, a mechanism used in a printing apparatus for depositing ink onto a printable substrate, or a droplet depositing mechanism. Those skilled in the art will appreciate that other means for depositing or applying ferrofluid onto the optical waveguide 102 may be implemented for use as the applicator 806.

In some embodiments, the personal care device 800 may comprise a fluid displacement device 808 for displacing at least a portion of the volume of the ferrofluid 302 from the optical waveguide 102. The fluid displacement device 808 may be configured to remove some or all of the ferrofluid from the optical waveguide 102, for example after the ferrofluid has been used to treat the optical waveguide. In some embodiments, the fluid displacement device 808 may comprise a fluid flow device, such as a device configured to direct fluid (e.g. air or water) towards the optical waveguide 102 and/or towards the volume of ferrofluid to remove excess ferrofluid from the optical waveguide.

It will be appreciated that, while the magnetic field movement mechanism 804, the applicator 806 and the fluid displacement device 808 are shown in Fig. 9 to be components of the head portion 110 of the personal care device 800, those features may, in other embodiments, be components of another part of the personal care device, and/or positioned elsewhere in the personal care device, such as in the handle portion 108.

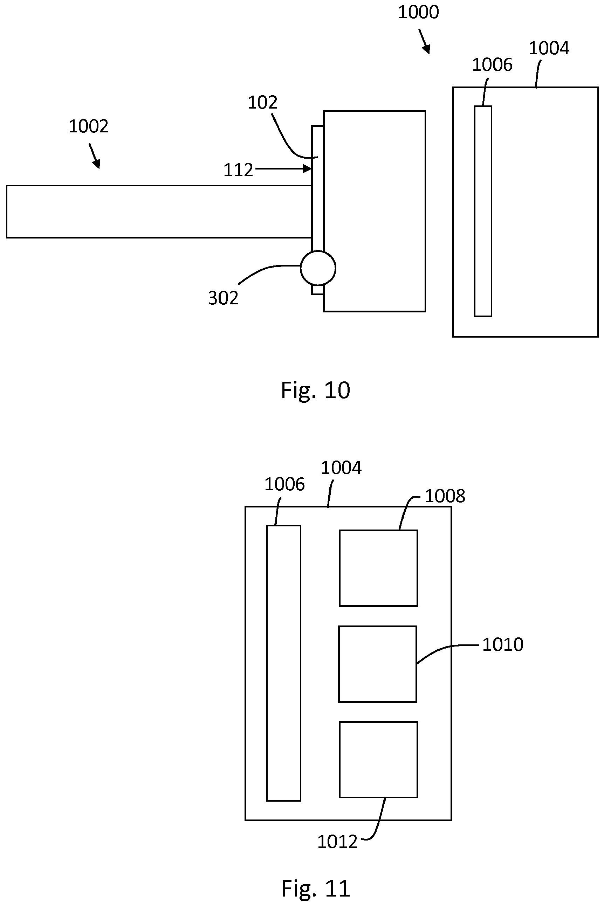

In the embodiment shown in Fig. 8, the magnetic field source 802 is located in the personal care device 800. However, in other embodiments, the magnetic field source 802 may be located remote from the personal care device. Fig. 10 is a schematic illustration of an example of a system 1000, such as a personal care system. The system 1000 comprises a personal care device 1002 and a docking unit 1004. The personal care device 1002 comprises an optical waveguide 102 having a sidewall, wherein a portion of the sidewall forms a cutting face 112 for contacting hair. The personal care device 1002 may include a head portion 110 and a handle portion 108. The docking unit 1004 is for receiving at least a portion of the personal care device. For example, the docking unit 1004 may be configured to receive the head portion 110 of the personal care device 1002 in a suitably sized and shaped receiving portion (not shown). The docking unit 1004 comprises a magnetic field generator 1006 for generating a magnetic field to engage a volume of ferrofluid 302 disposed on the optical waveguide 102 of the personal care device 1002. The magnetic field generator 1006 may be the same, or similar to the magnetic field source 802 discussed above.

A user of the system 1000 may use the personal care device 1002 to perform a personal care activity, such as trimming facial hair. Once the personal care activity has been completed, the user may place the personal care device 1002 in or on the docking unit 1004 to treat (e.g. clean) the optical waveguide 102. In some embodiments, a user may initiate treatment of the optical waveguide 102 manually, for example by pressing a button. In other examples, treatment of the optical waveguide 102 may commence automatically upon detection that personal care device 1002 has been docked.

A volume of ferrofluid 302 may be applied to the optical waveguide 102 using any of the method described herein. The magnetic field generator 1006 may be configured of controlled to generate a magnetic field which engages and interacts with the volume of ferrofluid, thereby causing the ferrofluid to move along the optical waveguide 102. The ferrofluid may treat the optical waveguide 102 in the various ways discussed herein. For example, the ferrofluid may cause debris (e.g. pieces of cut hair) to be removed from the optical waveguide 102, and a film or coating may be formed on the optical waveguide, which may improve the effectiveness of hair cutting.

Fig. 11 is a schematic illustration of a further example of the system 1000. In Fig. 11, only the docking unit 1004 is shown. The docking unit 1004 includes the magnetic field generator 1006. According to various embodiments, as shown in Fig. 11, the docking unit 1004 may also include additional functionality, as discussed herein.

In some embodiments, the docking unit 1004 may comprise a magnetic field movement mechanism 1008 for causing the magnetic field to move relative to the optical waveguide 102. The magnetic field movement mechanism 1008 may be the same as, or similar to, the magnetic field movement mechanism 804 discussed above. In this embodiment, however, the mechanism 1008 is located in the docking unit 1004 rather than in the personal care device. The magnetic field movement mechanism 1008 may, in some embodiments, move the magnetic field generator 1006.

The docking unit 1004 may, in some embodiments, comprise an applicator 1010 for applying a volume of ferrofluid to the optical waveguide 102. The applicator 1010 may be the same as, or similar to, the applicator 806 discussed above. In this embodiment, however, the mechanism 1008 is located in the docking unit 1004 rather than in the personal care device.

In some embodiments, the docking unit 1004 may comprise a fluid displacement device 1012 for displacing at least a portion of the volume of the ferrofluid from the optical waveguide 102. The fluid displacement device 1012 may be the same as, or similar to, the fluid displacement device 808 discussed above. In this embodiment, however, the fluid displacement device 1012 is located in the docking unit 1004 rather than in the personal care device.

It will be appreciated by those skilled in the art that the personal care devices and/or the systems disclosed herein may comprise one or more additional components not discussed herein. For example, the devices and/or systems may include a power source for supplying power to one or more other components.

The methods, devices and systems described herein provide an effective means for treating an optical waveguide of a personal care device in manner which reduces the likelihood of damaging the optical waveguide, and which can improve the hair cutting effectiveness of the device.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

## Claims

1. A method (500) of treating an optical waveguide (102), the method comprising:
Applying (502) the volume of ferrofluid (302) to the optical waveguide; and
applying (504) a magnetic field to the volume of ferrofluid to move the volume of ferrofluid at least partially along a length of the optical waveguide.

2. A method according to claim 1, wherein applying a magnetic field comprises applying a magnetic field generated by at least one of a permanent magnet and an electromagnet.

3. A method according to claim 1 or claim 2, wherein the ferrofluid comprises a binder fluid.

4. A method according to any of the preceding claims, wherein the ferrofluid comprises a chemical for providing a cleaning effect to the optical waveguide (102).

5. A method according to any of the preceding claims, wherein the ferrofluid comprises an additive for causing an abrasive effect when the additive is moved into contact with the optical waveguide (102).

6. A method according to any of the preceding claims, further comprising:
causing (602) the magnetic field to move at least one of longitudinally relative to the optical waveguide (102) and laterally relative to the optical waveguide.

7. A method according to any of the preceding claims, further comprising:
displacing (702) at least a portion of the volume of ferrofluid from the optical waveguide (102).

8. A personal care device (800) comprising:
an optical waveguide (102) having a sidewall, wherein a portion of the sidewall forms a cutting face (112) for contacting hair; and
a magnetic field source (802) configured to generate a magnetic field for engaging a volume of ferrofluid (302) present on the optical waveguide.

9. A personal care device according to claim 8, further comprising:
a magnetic field movement mechanism (804) for causing the magnetic field to move relative to the optical waveguide (102).

10. A personal care device according to claim 8 or claim 9, further comprising:
an applicator (806) for applying a volume of ferrofluid to the optical waveguide (102).

11. A personal care device according to any of claims 8 to 10, further comprising: a fluid displacement device (808) for displacing at least a portion of the volume of the ferrofluid from the optical waveguide (102).

12. A system (1000) comprising:
a personal care device (1002), the personal care device comprising an optical waveguide (102) having a sidewall, wherein a portion of the sidewall forms a cutting face for contacting hair; and
a docking unit (1004) for receiving at least a portion of the personal care device, the docking unit comprising:
a magnetic field generator (1006) for generating a magnetic field to engage a volume of ferrofluid (302) disposed on the optical waveguide of the personal care device.

13. A system according to claim 12, wherein the docking unit further comprises:
a magnetic field movement mechanism (1008) for causing the magnetic field to move relative to the optical waveguide (102).

14. A system according to claim 12 or claim 13, wherein the docking unit further comprises:
an applicator (1010) for applying a volume of ferrofluid to the optical waveguide (102).

15. A system according to any of claims 11 to 14, wherein the docking unit further comprises:
a fluid displacement device (1012) for displacing at least a portion of the volume of the ferrofluid from the optical waveguide (102).
